# EUROPEAN PATENT APPLICATION

(11) **EP 1 688 364 A1**
(43) Date of publication of application: **09.08.2006**
(21) Application number: 05112669.6
(22) Date of filing: 21.12.2005
(51) Int. Cl.: B65D 75/58, A61F 15/00

(54) **Flexible package for absorbent articles, having dual opening system**

(30) Priority: 04.02.2005 EP 05002438
(71) Applicant: THE PROCTER & GAMBLE COMPANY, Cincinnati, Ohio 45202 (US)
(72) Inventor: Somma, Emma, D-60318, Frankfurt (DE); Kohlweyer, Christian, 61118, Bad Vilbel (DE); Ulas, Ibrahim, D-61440, Oberursel (DE)
(74) Representative: Kremer, Véronique Marie Joséphine

(57) **Abstract**

The present invention is related to a flexible packaging for a multiplicity of absorbent articles. The package includes a refastenable flap (2) as first opening means and underneath the flap a secondary opening means (3). The package provides improved hygienic protection of the absorbent articles remaining in the package after its opening.

## Description

### FIELD OF THE INVENTION

The present invention is related to a flexible packaging for a multiplicity of absorbent articles. The package includes a refastenable flap as first opening means and underneath the flap a secondary opening means. The package provides improved hygienic protection of the absorbent articles remaining in the package after its opening.

### BACKGROUND OF THE INVENTION

Flexible packages for absorbent articles are known own in the art. Due to the high hygienic requirements of sanitary absorbent articles, like sanitary napkins, panty liners, incontinence pads or diapers, it is conventional to package e.g. sanitary napkins in a folded flat configuration into a flexible film individually, such as the case with ALWAYS® sanitary napkins as marketed by the Procter & Gamble Company. This requires a high amount of package material. Therefore attempts have been made to develop packages for a multitude of absorbent articles in order to save material. Examples are WO-A-02/39943 or WO-A-02/085271, disclosing packages for a stack of absorbent articles, both having a reclosable flap. However, packages of this kind are not completely sealed against dust, moisture and other contamination. Therefore, especially while those packages are in the warehouse or on the shelves of a retailer, the content of such a package is to a certain extend exposed to contamination. The contamination of the stored absorbent articles can be prevented by individually wrapping them. However, this again consumes extra material.

Therefore, a need exists to provide a package for a multitude of absorbent articles, which is reclosable and which protects the articles contained therein from contamination during storage and shelf life without the need of further packaging or wrapping material.

Beneficially, such a package should allow convenient opening and easy dispensing of the products contained therein.

### SUMMARY OF THE INVENTION

The present invention addresses the above problem by providing a package according to claim 1. As outlined, the package of the present invention has a dual opening system. A reclosable outer flap as primary opening means is connected to the main package body and can be moved between an open and a closed configuration. When the flap is opened the secondary opening means is exposed. When closed, the secondary opening means seals the main package body against the environment in a dust-proof manner and also provides tamper-evidence. Once opened, the secondary opening means is arranged to provide an opening in the main package body, through which the articles contained therein can be removed.

In one preferred embodiment the opening in the main package portion provided by the secondary opening means is essentially linear. This essentially linear shape allows an absorbent article to be removed, however also provides only a small area for allowing dust and other contamination access the main package body. Further, by closing the reclosable flap the essentially linear opening will be covered by the flap, which further aids preventing contaminants from entering the package.

In another preferred embodiment the opening provided by the secondary opening means is 2-dimensional and thus allows convenient dispensing of the articles contained in the package. In this embodiment the secondary opening means is provided by a tab or panel, which can be pulled off by the user to expose the opening. The opening is beneficially provided as a cut-out in the material of the package.

The opening created in the main package body by opening the secondary opening means can be covered by closing the flap. By this a hygienically satisfactory storage of absorbent articles is provided even when the user has opened the package.

Thus, a package for a plurality of absorbent articles is provided, which obviates the need for wrapping the individual articles by providing a high degree of protection, both during storage and shelf live and during use by a consumer.

### BRIEF DESCRIPTION OF THE DRAWINGS

- Fig. 1: illustrates a package (1) of the present invention with the flap (2) is in its closed configuration.
- Fig. 2: illustrates a package (1) of the present invention with the flap (2) in its open configuration. The secondary opening means (3) is exposed.
- Fig. 3: illustrates a package (1) of the present invention with the flap (2) in its open configuration and the secondary opening means (3) partially opened, thereby exposing the essentially linear opening (6). It is visible that two stacks of absorbent articles are contained in the main package body (5).

### DETAILED DESCRIPTION OF THE INVENTION

The term 'absorbent article' is used herein in a very broad sense, including any article suitable for feminine protection, being able to receive and/or absorb and/or contain and/or retain body fluids. The absorbent article of the present invention typically comprises a structure having a fluid pervious topsheet as the wearer-facing layer, a fluid impervious backsheet that is preferably water vapour and/or gas pervious as the garment-facing layer and an absorbent core element comprised there between. Particularly preferred absorbent articles in the context of the present invention are disposable absorbent articles. Preferred disposable absorbent articles according to the present invention are disposable absorbent articles for feminine protection like incontinence pads, sanitary napkins, tampons, breast pads or panty liners. Other suitable absorbent articles are incontinence pads and perspiration pads. Particularly preferred are substantially flat absorbent articles for ease of stacking. The absorbent articles can be individually packaged or can alternatively be without individual packaging. The absorbent articles in the package can be arranged in one or more stacks in any suitable manner.

The term 'disposable' is used herein to describe absorbent articles, which are not intended to be laundered or otherwise restored or reused as an article (i.e. they are intended to be discarded after a single use and preferably to be recycled, composted or otherwise disposed of in an environmentally compatible manner).

The term 'stack' herein refers to a multitude of articles being arranged behind one another or on top of each other in an essentially coextensive manner. The package of the present invention contains at least one, and optionally multiple, stacks of absorbent articles.

The term 'dust proof' herein means that the package is sealed such that solid contaminants cannot access its interior. This implies that the package is in one preferred embodiment free of perforations and the like, because these would allow solid contaminants access the package. Also, tamper proofness is a consumer benefit especially for hygiene products. A sufficient degree of dust proofness can also be achieved with the secondary opening means comprising perforations, because these perforations will be covered by the primary opening means, i.e. the flap, and thus dust and other contaminants will be significantly hindered from accessing the interior of the package.

The main package body is made from suitable material, which offers the required degree of hygienic protection to the articles stored therein. Preferably, the package of the present invention is made of flexible polymeric film material. Suitable materials are PE (polyethylene) and derivatives like ethylen-vinylacetate-coploymers, polypropylene (PP), polyester, Polystyrol (PS), cellophane, polyvinylchloride (PVC), polyvinylalcohol (EVOH), polyvinylacetal (e.g. PVB), flouropolymers, polyethylenterephtalat (PET), Polycarbonat (PC), Polyamide (PA), Polyphenylenoxid, Polysulfon, Polyurethanes and the like. Also encompassed are biodegradable polymers based on aliphatic or aromatic polyesters, starch or similar materials that are suited for flexible film production. This also includes laminated or co-extruded material made from above materials. Particularly preferred are PE, PP, polyester, cellophane and PVC. Furthermore, suitable materials for making the main package body are materials comprising nonwovens and / or paper, provided they are substantially impervious to contaminants such as dust. Examples are laminates of nonwovens and polymeric films or silicone- or wax-treated paper. The main package body can also be made of more then one layer of material, such as an inner and an outer layer.

The package comprises a main package body and a flap. The main package body completely encloses the articles contained therein. The main package body can have any suitable size and shape. A preferred example has a substantially rectangular parallelepipedal shape. The main package body is provided with a secondary opening means, which, when opened, provides an opening in the main package body through which the articles can be conveniently removed. The opening typically has a length / width ratio of from 1 to 12.

In a preferred embodiment said opening in the main package body is essentially linear. 'Essentially linear opening' herein means that the opening has a length much larger than its width. In the package of the present invention the essentially linear opening has a length/width ratio in the range of from 5 to 9. The linearity of the opening provided by the secondary opening means translates in a very low area of the actual opening compared to a circular or ellipsoidal opening. This again means a smaller area for contaminants to enter the package after opening the secondary opening means. When closed the secondary opening means seals the articles contained in the main package body in a dust proof manner. Suitable means for providing the secondary opening means are tear tapes, zip-lock closure means, adhesive tapes covering the essentially linear opening, and weakness- or score lines in the material of the main package body.

In another preferred embodiment herein said opening in the main package body is 2-dimensional for allowing convenient dispensing of the articles contained in the package. The opening can have any size and shape, as long as it is fully covered by the primary opening means, i.e. the flap. Exemplary, non-limiting shapes are circular, ellipsoidal, rectangular, square or triangular.

The flap is connected to the main package body along a line of connection. 'Connected' herein does not imply that the flap has to be a separate piece of material, which needs to be joined to the main package body. Thus, 'line of connection' is to be interpreted as the line along which the flap extends away from the main package body when the flap is in its open configuration. It is also within the scope of the present invention that main package body and flap are made of one interconnected piece of material. The flap can have any suitable size and shape. Preferred herein is a configuration, which has a width at the line of connection identical to the width of the main package body along said line of connection. The flap can be pivoted between an open and a closed configuration. In the closed configuration the free end of the flap can be secured to the main package body by an adhesive tape, a Velcro-type hook-loop fastener or the like. The line of connection between the main package body and the flap is arranged such that, when the flap is in its open position, it exposes the opening provided by the secondary opening means, and when the flap is in its closed position, it covers the opening provided by the secondary opening means in the main package body. This is especially advantageous when the secondary opening means is opened because the flap then covers the opening created thereby and thus aids the hygienic protection of the articles still contained in the main package body.

The flap can be joined or substantially non-joined to the secondary opening means. In the first case opening of the flap and opening of the secondary opening means are essentially independent from each other. In the latter case opening of both primary (flap) and secondary opening means can be coupled.

The flap can be joined to the secondary opening means such that when the flap is opened also the secondary opening means opens and exposes the opening in the main package body. This can be facilitated e.g. by heat or pressure bonds or by adhesives.

In other embodiments of the present invention the flap is arranged to initially not cover the secondary opening means prior to use. This for instance refers to the shelf life of the package. The flap is then initially not pivoted over the secondary opening means but into the opposite direction, towards the backside of the package. When the package is then used, i.e. opened, the flap is de-joined from the backside of the package and pivoted over the opening generated by opening the secondary opening means. The free end of the flap can optionally be secured in this position by the means disclosed above for this purpose. Of course, when an adhesive is used therefore a protective cover like a release liner of conventional manner is needed to preserve the adhesive in functional state over longer time intervals.

The means for securing the flap in its closed position can be coloured for serving as identification means. This is particularly relevant in case the flap is transparent or translucent.

In an embodiment where the main package body is made of two layers of material the flap is an integral part of the outer layer. For instance, the flap can be formed by lines of perforations in the outer layer of material, which are broken by the user when opening the outer layer. By the opening generated thereby, the inner layer is exposed in the region of the opening. The secondary opening means is a perforation in the exposed area of the inner layer of material.

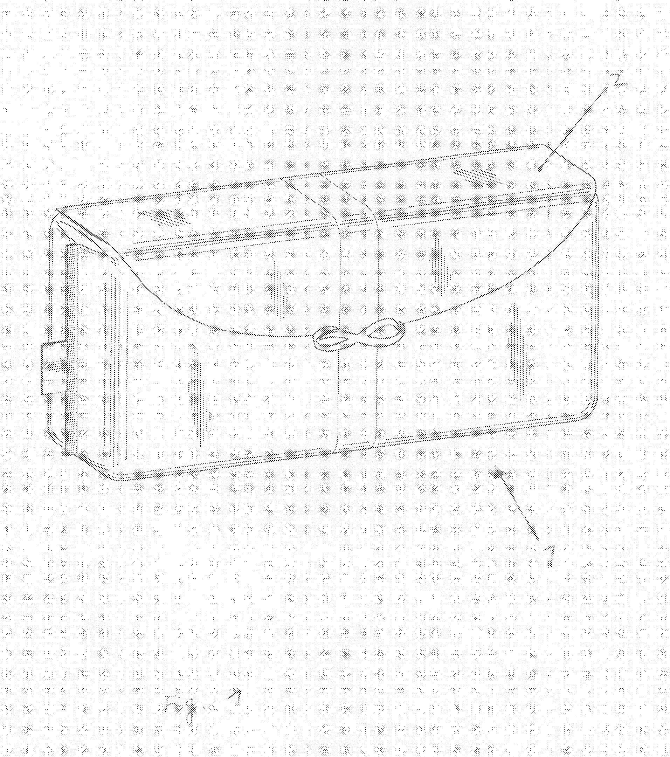

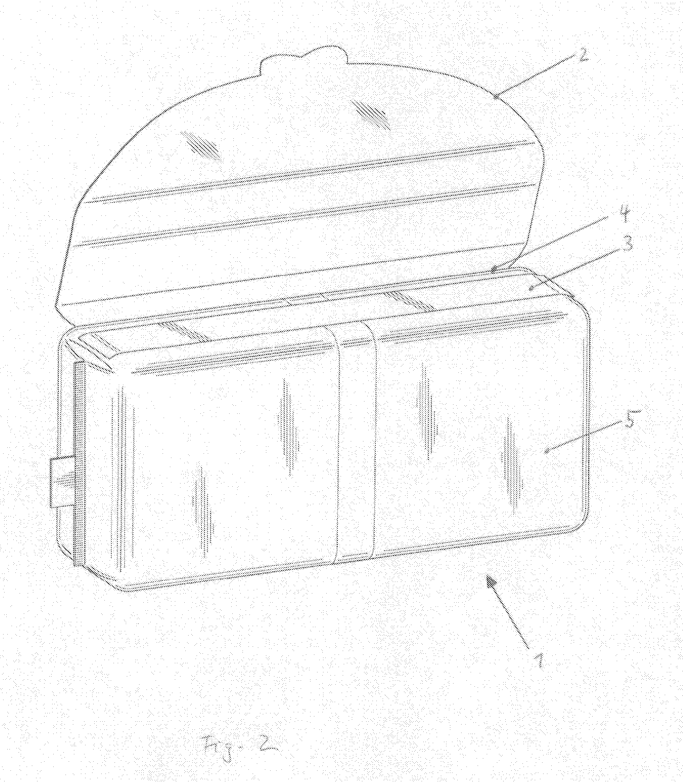

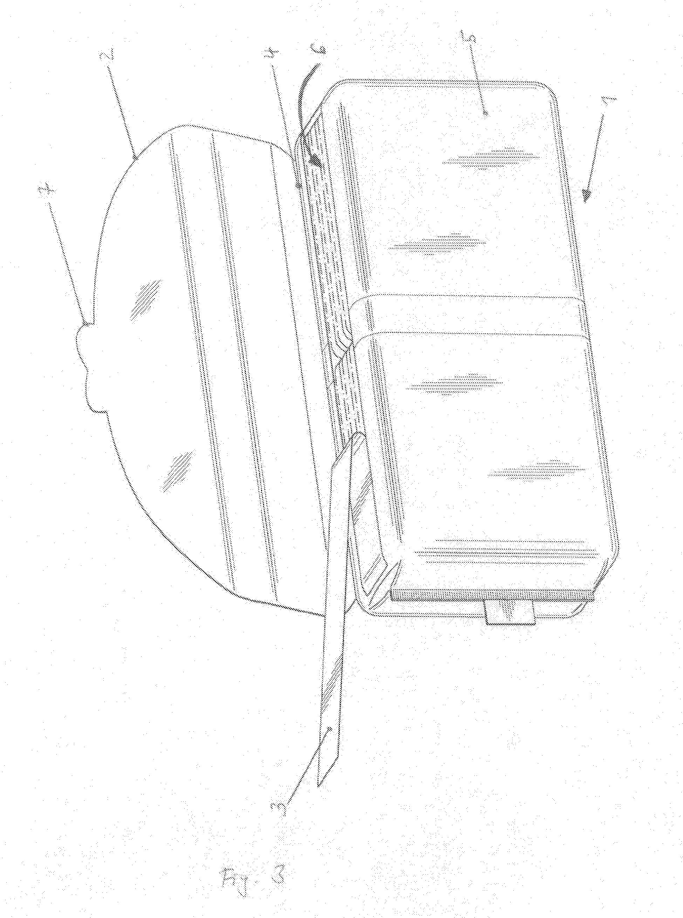

A preferred embodiment herein is illustrated in Figures 1-3. Figure 1 illustrates the package in completely closed state, whereas Figure 2 illustrates the package with the flap (2) opened to expose the secondary opening means (3). Figure 3 then illustrates the package with the secondary opening means (3) partially opened. The main package body (1) has a substantially parallelepipedal shape. The line of connection (4) of the flap (2) to the main package body (1) extends parallel to a side edge of the main package body (5) and has the same length of said edge. Between said line of connection (4) and an adjacent side edge of the main package body (5) the secondary opening means (3) is extending. The secondary opening means (3) is executed as a tear tape (3) having a free end for grasping. Said tear tape (3) is arranged close and parallel to said line of connection (4) such that the flap (2), when closed, completely covers the tear tape (3). The flap (2) has a semi-circular shape with a piece of adhesive tape (7) connected to its free end for securing to the main package body (5). The absorbent articles contained in the package are arranged in two stacks.

All documents cited in the Detailed Description of the Invention are, in relevant part, incorporated herein by reference; the citation of any document is not to be construed as an admission that it is prior art with respect to the present invention.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A package (1) for a multiplicity of absorbent articles arranged in at least one stack, said package (1) comprising a main package body (5) and a reclosable flap (2), said main package body (5) completely encloses said stack, said reclosable flap (2) being hingedly connected to said main package body (5) along a line of connection (4), wherein said flap (2) can be pivoted between an open and a closed configuration,
**characterized in that** said package (1) further comprises a secondary opening means (3) being capable of providing an opening (6) in said main package body (5), wherein said secondary opening means (3) is arranged in relation to said line of connection (4) such that said flap (2) in said closed configuration covers said opening (6) provided by said secondary opening means (3).

2. The package (1) of any of the preceding claims, wherein said opening (6) has a length / width ratio of from 1 to 12.

3. The package (1) of claim 2, wherein said opening (6) has a length / width ratio of from 5 to 9.

4. The package (1) of claim 3, wherein said opening (6) provided by said secondary opening means (3) is essentially linear.

5. The package (1) of any of the preceding claims, wherein said reclosable flap (2) can be reclosed by an adhesive tape (7) or a hook and loop mechanical fastener.

6. The package (1) of any of the preceding claims, wherein said package (1) is made from a flexible polymeric film material such as polyethylene, polypropylene, cellophane, polyester or polyvinyl chloride.

7. The package (1) of any of the preceding claims, wherein said secondary opening means (3) is provided by a tear tape or a zip-lock closure or a weakness or score line.

8. The package (1) of any of the preceding claims, wherein said absorbent articles are selected from sanitary napkins, panty liners, tampons, incontinence pads, breast pads and perspiration pads.
